# EUROPÄISCHE PATENTANMELDUNG

(11) **EP 1 021 988 A1**
(43) Veröffentlichungstag der Anmeldung: **26.07.2000**
(21) Anmeldenummer: 00101117.0
(22) Anmeldetag: 21.01.2000
(51) Int. Cl.: A61B 8/12

(54) **Katheter zum Einführen in Blutbahnen**

(30) Priorität: 25.01.1999 DE 29901179 U
(71) Anmelder: SCHREGEL, WERNER, PROF. DR. MED., 47802 KREFELD (DE)
(72) Erfinder: SCHREGEL, WERNER, PROF. DR. MED., 47802 KREFELD (DE)
(74) Vertreter: DR. STARK & PARTNER

(57) **Zusammenfassung**

Die Erfindung betrifft einen Katheter zum Einführen in Blutbahnen bestehend aus einem im Wesentlichen rohrförmigen Instrument mit zumindest einem Infusions- bzw. Absaugkanal, der im Endbereich zumindest eine Öffnung aufweist. Um einen Katheter insbesondere zur Prävention und Therapie von Luftembolien anzugeben, der einfacher ohne weitere Hilfsmittel plaziert werden kann, soll in dem Katheter wenigstens eine Messeinrichtung zur Detektion von Gasen in Flüssigkeiten vorgesehen sein.

## Beschreibung

Die Erfindung betrifft einen Katheter zum Einführen in Blutbahnen bestehend aus einem im Wesentlichen rohrförmigen Instrument mit zumindest einem Infusions- bzw. Absaugkanal, der im Endbereich zumindest eine Öffnung aufweist.

Mittels derartiger Katheter können auf der einen Seite Flüssigkeiten in Körperhohlorgane wie z. B. Blutbahnen injiziert oder Proben aus Körperhohlorganen entnommen werden. Derartige Katheter werden aber auch bei Operationen insbesondere bei Operationen in (halb)sitzender Lagerung des Patienten zur Prävention und Therapie von Luftembolien eingesetzt. Luftembolien können auftreten, wenn venöse Gefäße geöffnet werden und ein negativer Venendruck infolge eines großen Blutverlustes z. B. bei Transplantationen herrscht. Hierbei kann es zu einem Ansaugen von Luft in das zentrale Venensystem und somit zu einer Ansammlung größerer Luftmengen kommen, so dass der Bluteinstrom zum Herzen blockiert werden kann.

Zum Absaugen derartiger Luftmengen im zentralen Venensystem wird der Katheter, der zumindest eine Öffnung aufweist, in der oberen Hohlvene am Eingang zum rechten Herzvorhof plaziert. Bei Verwendung eines Katheters mit mehreren seitlichen Öffnungen wird die Spitze des Katheters eher in der oberen Hälfte des rechten Herzvorhofes plaziert. Über die Öffnung(en) des Katheters wird während einer Operation die eventuell in das zentrale Venensystem eingedrungene Luft abgesaugt und so eine Luftembolie verhindert. Als Nachteil erweist sich, dass derartige Katheter zur Vermeidung von Luftembolien genau plaziert werden müssen, jedoch die Plazierung nicht einfach ist, da die Lage des Katheters nicht genau von außen bestimmbar ist.

Zum anderen bedarf es neben dem Katheter eines weiteren Instruments, um eventuell in das zentrale Venensystem eingedrungene Luft zu detektieren, die dann mit dem Katheter abgesaugt werden kann.

Sofern die transoesophageale Echokardiographie angewandt wird, wird ein flexibles Rohr mit einer endseitigen Ultraschalleinrichtung in die Speiseröhre des Patienten eingeführt. Nachteilig erweist sich bei dieser Methode, dass die hierzu erforderlichen Apparate sehr aufwendig sind und zum anderen der Patient nicht nur während der Operation, sondern auch mehrere Stunden danach durch einen zusätzlichen Arzt überwacht werden muss. Insofern ist diese Methode sehr kostenintensiv.

Bei einem anderen Verfahren zur Entdeckung von Luftembolien wird eine Dopplersonde von außen im Bereich des Herzens auf dem Körper des Patienten fixiert. Die damit erzielten Messergebnisse sind teilweise recht ungenau, da zum einen der Abstand zwischen der auf der Haut fixierten Dopplersonde und dem Herzen groß ist und zum anderen die Messergebnisse durch im Operationstrakt zur Blutstillung benutzte Geräte wie z. B. Elektrokauter beeinflusst werden können. Darüber hinaus muss die Dopplersonde genau plaziert sein. Bei Patienten mit Lungenvergrößerungen, beispielsweise infolge von Bronchitis oder Asthma, ist dieses Verfahren überhaupt nicht anwendbar.

Aufgabe der Erfindung ist es, einen Katheter insbesondere zur Prävention und Therapie von Luftembolien anzugeben, der die vorgenannten Nachteile vermeidet.

Diese Aufgabe wird dadurch gelöst, dass in dem Katheter wenigstens eine Messeinrichtung zur Detektion von Gasen in Flüssigkeiten vorgesehen ist. Durch die Integration der Messeinrichtung in den Katheter kann der Katheter nicht nur zum Absaugen von Luftmengen - sofern der Katheter zur Prävention und Therapie von Luftembolien bei Operationen eingesetzt wird -, sondern ebenfalls zur Detektion von Luftmengen in Flüssigkeiten, wie z. B. Blut, verwendet werden. Auch ist die Plazierung des Katheters durch die integrierte Messeinrichtung leichter. Da der Katheter mit der Messeinrichtung von der zu überwachenden Flüssigkeit direkt umspült wird und somit in unmittelbarem Kontakt mit der zu überwachenden Flüssigkeit steht, sind Störungen, wie sie bei herkömmlichen Verfahren auftreten, auf ein Minimum reduziert. Da lediglich die Messeinrichtung(en) in dem Katheter integriert ist(sind), während die anderen für das Absaugen bzw. Injizieren und Detektion erforderliche Bauteile wie Steuer- oder Absaugeinrichtung außerhalb des Katheters angeordnet sind, weisen derartige Katheter geringste Durchmesser auf.

Zweckmäßigerweise kann die Messeinrichtung eine Ultraschallsonde sein, die wenigstens aus einem Empfänger und einem Sender, die winklig zueinander angeordnet sind, bestehen kann. Bei einer anderen Ausführungsform kann eine in mehrere Richtungen, insbesondere zirkulär abstrahlende Messeinrichtung vorhanden sein. Es ist aber auch durchaus eine Kombination aus versetzt zueinander angeordneten Messeinrichtungen und einer zirkulär abstrahlenden Messeinrichtung möglich. Derartige Sonden machen sich den sogenannten "Doppler-Effekt" zunutze, bei dem die Frequenzänderung zwischen den von dem Empfänger ausgestrahlten und von der Flüssigkeit bzw. Luftblasen reflektierten und vom Empfänger empfangenen Wellen ermittelt wird. Strömt beispielsweise eine in einer Blutbahn befindliche Luftblase entlang der Ultraschallsonde, so verändert sich die Frequenz der von der Luftblase reflektierten Wellen. Diese Frequenzänderung wird dann beispielsweise akustisch oder optisch angezeigt, so dass dann die Luftblase durch die Öffnungen des Katheters abgesaugt werden kann.

Damit die Messeinrichtung wiederverwendbar ist und nicht mit dem Blut - sofern der Katheter in einer Blutbahn eingeführt ist - in Verbindung kommt, kann wenigstens ein endseitig geschlossener Einführkanal vorgesehen sein. Die Messeinrichtung wird hierzu vor der Operation in den endseitig geschlossenen Einführkanal geschoben, so dass ein Kontakt mit der zu überwachenden Flüssigkeit vermieden wird. Der so mit der Messeinrichtung versehene Katheter wird dann in die betreffende Blutbahn eingeführt.

Bei einer anderen Ausführungsform der Erfindung kann beidseits des Infusions- bzw. Absaugkanals je ein geschlossener Einführkanal vorgesehen sein, Zweckmäßigerweise können dabei mehrere Messeinrichtungen versetzt zueinander angeordnet sein. Hierdurch ist gewährleistet, dass durch Überlappen der Abstrahlwinkel das gesamte Gefäßvolumen erfasst wird, so dass Luftblasen in jedem Bereich des Gefäßvolumens detektiert werden können.

Dabei bietet es sich an, dass der Katheter endseitig und/oder seitlich Öffnungen aufweisen kann, um die detektierten Luftblasen absaugen zu können.

Bei einer besonders vorteilhaften Ausführung der Erfindung ist/sind die Messeinrichtung(en) im Abstand zu dem Ende des Katheters angeordnet, und der Katheter weist zumindest zwischen der/den Messeinrichtung(en) seitliche Öffnungen auf. Der Katheter wird dabei derart plaziert, dass sich die Messeinrichtung(en) im Bereich des Eingangs zum rechten Herzvorhof befindet (befinden), so dass der Teil des Katheters mit den Öffnungen im Herzvorhof plaziert ist. Sofern durch die Messeinrichtung(en) in dem aus der Blutbahn über den rechten Herzvorhof in das Herz einströmenden Blut Luftblasen detektiert werden, können diese unmittelbar über die in Strömungsrichtung folgenden Öffnungen des Katheters abgesaugt werden.

Im folgenden wird ein in den Zeichnungen dargestelltes Ausführungsbeispiel der Erfindung erläutert. Es zeigen:
- Fig. 1: eine Seitenansicht auf einen erfindungsgemäßen Katheter,
- Fig. 2: einen Schnitt durch einen Katheter mit einem Infusions- bzw. Absaugkanal und einer integrierten Messeinrichtung,
- Fig. 3: einen Schnitt durch einen Katheter mit einem Infusions- bzw. Absaugkanal und einem endseitig geschlossenen Einführkanal, in dem eine Messeinrichtung eingeführt ist, und
- Fig. 4: einen Schnitt durch einen Katheter mit einem Infusions- bzw. Absaugkanal und zwei endseitig geschlossenen Einführkanälen, in denen jeweils eine Messeinrichtung eingeführt ist.

In sämtlichen Zeichnungen werden für gleiche bzw. gleichartige Bauteile die gleichen Bezugszeichen verwendet.

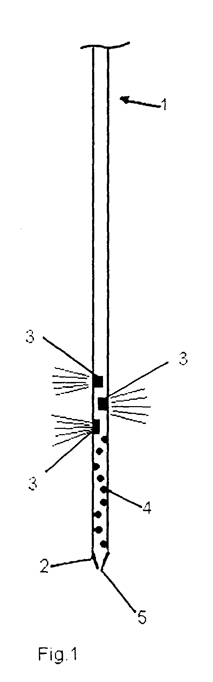

Fig. 1 zeigt einen Katheter 1, der aus einem im Wesentlichen rohrförmigen Instrument besteht. Der Katheter 1 wird in eine nicht dargestellte Blutbahn eingeführt, wobei das eine Ende 2, mit dem der Katheter 1 in die Blutbahn eingeführt wird, kegelstumpfartig ausgebildet ist.

Im Abstand zum Ende 2 des Katheters 1 sind Messeinrichtungen 3 zur Detektion von Gasen, wie z. B. Luftmengen, in Flüssigkeiten vorgesehen. Die Messeinrichtungen 3 sind vorteilhafterweise so angeordnet, dass die Abstrahlwinkel sich überlappen, damit das ganze Gefäßvolumen der Blutbahn überwacht werden kann.

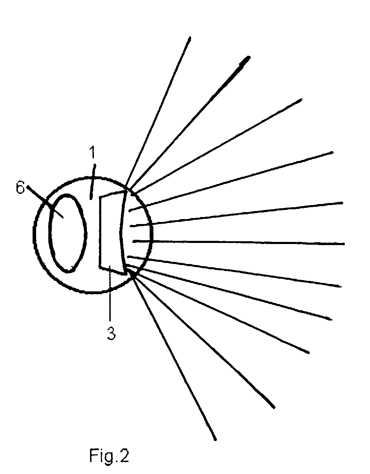

Zwischen den Messeinrichtungen 3 und dem Ende 2 des Katheters 1 sind seitliche Öffnungen 4 sowie im Bereich des Endes 2 eine endseitige Öffnung 5 vorgesehen, die mit einem, wie in Fig. 2 dargestellt, Infusions- bzw. Absaugkanal 6 verbunden sind.

Das andere Ende des Katheters 1 ist mit einer herkömmlichen nicht dargestellten Infusions- bzw. Absaugeinrichtung für den Infusions- bzw. Absaugkanal 6 sowie einer ebenfalls nicht dargestellten Steuer- und Anzeigeeinrichtung für die Messeinrichtungen 3 verbunden.

Der Katheter 1 wird vor einem operativen Eingriff mit dem Ende 2 in eine nicht dargestellte Blutbahn eingeführt und dabei so plaziert, dass der Katheter 1 mit den Messeinrichtungen 3 am Eingang zum rechten Herzvorhof angeordnet ist, da hier das Venensystem den geringsten Durchmesser aufweist und daher im Blut befindliche Luftmengen am besten ermittelt werden können. Die seitlichen Öffnungen 4 bzw. die endseitige Öffnung 5 des Katheters 1 befinden sich dabei im rechten Herzvorhof. Wird nun mittels der Messeinrichtungen 3 eine Luftblase detektiert, so kann diese dann über die in Strömungsrichtung folgenden seitlichen Öffnungen 4 bzw. die endseitige Öffnung 5 abgesaugt werden.

Fig. 2 zeigt eine Variante des erfindungsgemäßen Katheters 1, bei der die Messeinrichtung 3 fest in dem Katheter 1 integriert ist. Deutlich zu erkennen ist, dass die Messeinrichtung 3 aus einem Empfänger und einem Sender besteht, die winklig zueinander angeordnet sind. Die von dem Sender ausgestrahlten Wellen werden von der Flüssigkeit bzw. eventuell vorhandenen Luftblasen reflektiert und vom Empfänger wieder empfangen. Dabei treten je nach dem reflektierten Medium, d. h. Flüssigkeit oder Luftblasen, Unterschiede in der Frequenz der gesendeten und empfangenen Wellen auf, so dass mittels dieser Technik Luftblasen detektiert werden können. Da der Katheter 1 von den Flüssigkeiten direkt umströmt wird, können selbst Luftblasen mit geringen Durchmessern gemessen werden. Die Messeinrichtung 3 befindet sich bei dieser Ausführungsform in der einen Hälfte des Katheters 1, während in der anderen Hälfte der Infusions- bzw. Absaugkanal 6 vorgesehen ist.

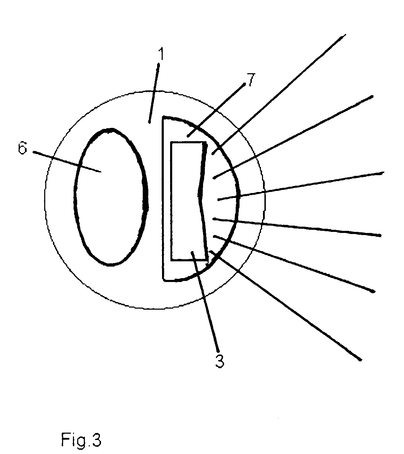

Fig. 3 zeigt eine Ausführungsform des Katheters 1, bei dem sich die Messeinrichtung 3 in einem in dem Katheter 1 angeordneten endseitigen geschlossenen Einführkanal 7 befindet. Da der Einführkanal 7 endseitig geschlossen ist, kommt die darin angeordnete und verschiebliche Messeinrichtung 3 nicht in Kontakt mit der zu überwachenden und den Katheter 1 umströmenden Flüssigkeit. Nach der Operation wird die Messeinrichtung 3 aus dem Einführkanal des Katheters 1 gezogen und kann in einen neuen Katheter 1 eingesetzt und somit wiederverwertet werden, ohne dass es einer aufwendigen Sterilisation bedarf. Auch hier ist der Infusions- bzw. Absaugkanal 6 in der anderen Hälfte des Katheters 1 angeordnet.

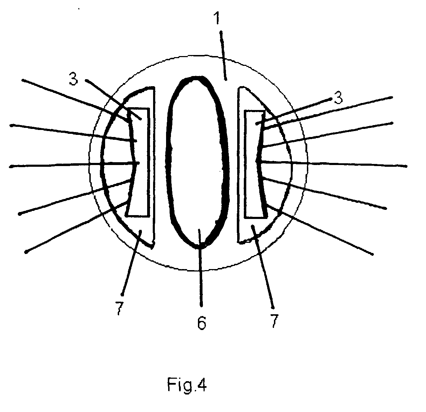

Bei der in Fig. 4 dargestellten Ausführungsform des Katheters 1 ist der Infusions- bzw. Absaugkanal 6 mittig angeordnet, wobei beidseits des Infusions- bzw. Absaugkanals 6 endseitig geschlossene Einführkanäle 7 vorgesehen sind. In diese Einführkanäle 7 sind Messeinrichtungen 3 eingeführt. Es bedarf keiner näheren Erläuterung, dass in jedem Einführkanal 7 mehrere Messeinrichtungen 3 vorgesehen sein können.

## Patentansprüche

1. Katheter (1) zum Einführen in Blutbahnen bestehend aus einem im Wesentlichen rohrförmigen Instrument mit zumindest einem Infusions- bzw. Absaugkanal (6), der im Endbereich zumindest eine Öffnung (4, 5) aufweist, dadurch gekennzeichnet, dass in dem Katheter (1) wenigstens eine Messeinrichtung (3) zur Detektion von Gasen in Flüssigkeiten vorgesehen ist.

2. Katheter (1) nach Anspruch 1, dadurch gekennzeichnet, dass die Messeinrichtung (3) eine Ultraschallsonde ist.

3. Katheter (1) nach Anspruch 2, dadurch gekennzeichnet, dass die Ultraschallsonde wenigstens aus einem Empfänger und einem Sender, die winklig zueinander angeordnet sind, besteht.

4. Katheter (1) nach einem der Ansprüche 1 bis 3, dadurch gekennzeichnet, dass wenigstens ein endseitig geschlossener Einführkanal (7) vorgesehen ist.

5. Katheter (1) nach Anspruch 4, dadurch gekennzeichnet, dass beidseits des Infusions- bzw. Absaugkanals (6) je ein geschlossener Einführkanal (7) vorgesehen ist.

6. Katheter (1) nach einem der Ansprüche 1 bis 5, dadurch gekennzeichnet, dass mehrere Messeinrichtungen (3) versetzt zueinander angeordnet sind.

7. Katheter (1) nach einem der Ansprüche 1 bis 6, dadurch gekennzeichnet, dass eine in mehrere Richtungen, insbesondere zirkulär abstrahlende Messeinrichtung (3) vorhanden ist.

8. Katheter (1) nach einem der Ansprüche 1 bis 7, dadurch gekennzeichnet, dass der Katheter (1) endseitig und/oder seitlich Öffnungen (5, 4) aufweist.

9. Katheter (1) nach einem der Ansprüche 1 bis 8, dadurch gekennzeichnet, dass die Messeinrichtung/en (3) im Abstand zu dem Ende (2) des Katheters (1) angeordnet ist/sind und der Katheter (1) zumindest zwischen der/den Messeinrichtung/en (3) seitliche Öffnungen (4) aufweist.
